# EUROPEAN PATENT APPLICATION

(11) **EP 4 108 652 A1**
(43) Date of publication of application: **28.12.2022**
(21) Application number: 21756863.3
(22) Date of filing: 08.01.2021
(51) Int. Cl.: C07C 29/04, C07C 31/08, C07B 61/00, B01J 23/30

(54) **METHOD FOR PRODUCING ALCOHOL**

(30) Priority: 20.02.2020 JP 2020027332
(71) Applicant: Showa Denko K.K., Tokyo 105-8518 (JP)
(72) Inventor: INOUE, Gen, Oita-shi, Oita 870-0189 (JP); KIMURA, Toshihiro, Oita-shi, Oita 870-0189 (JP); KOYANO, Masafumi, Oita-shi, Oita 870-0189 (JP)
(74) Representative: Strehl Schübel-Hopf & Partner
(86) International application number: PCT/JP2021/000565
(87) International publication number: WO 2021/166485

(57) **Abstract**

A method for producing an alcohol by hydrating an olefin using a heteropolyacid catalyst is provided, in which the catalyst can be stably used over a long period. The method is for producing an alcohol by supplying water and an olefin having 2-5 carbon atoms to a reactor and hydrating the olefin in a gas phase using a solid acid catalyst loaded with a heteropolyacid or a salt thereof, and is characterized in that a raw-material mixture to be supplied to the reactor has an aldehyde compound content of 70 mol ppm or less.

## Description

### TECHNICAL FIELD

The present invention relates to a method for producing an alcohol by a hydration reaction of an olefin using a heteropolyacid catalyst. The present invention is particularly suitable for the production of ethanol from ethylene.

### BACKGROUND ART

Industrial ethanol is an important industrial chemical product widely used as an organic solvent, an organic synthetic raw material, a disinfectant and an intermediate of chemicals. It is known that industrial ethanol can be obtained by a hydration reaction of ethylene in the presence of a liquid acid, such as sulfuric acid and sulfonic acid, a zeolite catalyst, a metal oxide catalyst including tungsten, niobium or tantalum, etc., a heteropolyacid, such as phosphotungstic acid and silicotungstic acid, or a solid acid catalyst in which phosphoric acid is supported on a silica carrier or a diatomite carrier. A hydration reaction of ethylene in a liquid phase using a liquid acid, such as sulfuric acid and sulfonic acid, as a catalyst requires a post-treatment of the acid used in the reaction, and in addition, is low in activity, so that industrial utilization thereof is limited.

On the other hand, a hydration reaction of ethylene using a solid acid catalyst of a carrier supported type can be carried out as a gas phase reaction, and there is an advantage that the separation of a reaction product and the catalyst is easy, and the reaction can be carried out under a high temperature condition which is advantageous in terms of reaction kinetics or a high pressure condition which is advantageous in terms of the theory of equilibrium. Regarding solid acid catalysts, many proposals have been made so far, and in particular, a gas phase reaction process using a catalyst in which phosphoric acid is supported on a carrier has been industrially carried out. However, in this industrial process using a catalyst in which phosphoric acid is supported on a carrier, an efflux of phosphoric acid, which is an active component, continuously occurs, and as a result, the activity and selectivity decrease. Thus, continuous supply of phosphoric acid is required. Further, periodic maintenance of a reactor and other equipment is necessary, and thus this costs a lot to maintain the reactor and other equipment, since the effused phosphoric acid corrodes the equipment. In addition, a phosphoric acid supported catalyst is physically and chemically deteriorated by contacting with water vapor. For that reason, when the phosphoric acid supported catalyst is used for a long period of time, the activity thereof decreases, and in some cases, carrier particles aggregate with each other to form a block, so that it is extremely difficult to replace and extract the catalyst. Therefore, a novel carrier and a supported catalyst have been developed to solve these problems.

As a catalyst for a hydration reaction of ethylene without a risk of an efflux of an acid, metal oxide catalysts are known, and a zeolite catalyst (Patent Literature 1), a metal oxide catalyst containing titanium oxide and tungsten oxide as essential components (Patent Literature 2), and a metal oxide catalyst containing tungsten and niobium as essential components (Patent Literature 3) are known. However, hydration reactions of ethylene using these metal oxide catalysts are less active than the case where a phosphoric acid catalyst is used, and the selectivity of the reaction is also low.

As another catalyst capable of avoiding an efflux of an acid, a solid acid catalyst in which a heteropolyacid is supported on a carrier is known. For example, a catalyst in which a heteropolyacid is supported on fumed silica obtained by a combustion method is disclosed as a supported catalyst for the production of ethanol by a hydration reaction of ethylene having improved performance (Patent Literature 4). As a method for improving the performance of a heteropolyacid supported catalyst, the use of a catalyst in which a heteropolyacid is supported on a clay carrier treated with a thermal acid has been proposed (Patent Literature 5). As a carrier of a supported catalyst suitable for a hydration reaction of an olefin, a silica carrier in which a pore volume, a specific surface area, and a pore diameter are specified is disclosed, and a catalyst for producing ethanol by a hydration reaction of ethylene using the silica carrier is also exemplified (Patent Literature 6).

### [CITATION LIST]

### [PATENT LITERATURE]

[PTL 1] JP H03-80136 B
[PTL 2] JP 3041414 B
[PTL 3] JP 2001-79395 A
[PTL 4] JP 3901233 B
[PTL 5] JP H08-225473 A
[PTL 6] JP 2003-190786 A

### SUMMARY OF INVENTION

### [TECHNICAL PROBLEM]

Although an attempt has been made to improve the performance of a heteropolyacid supported catalyst in this way, it is desirable that a heteropolyacid supported catalyst can be stably used for a long period of time from an economic viewpoint, since the price of a heteropolyacid is expensive as compared with phosphoric acid. In particular, in the case that impurities are contained in a raw material, when a catalyst is used for a long period of time, the impurities or compounds to which the impurities are converted may accumulate on the catalyst surface, which would adversely affect the stable use of the catalyst. However, in general, the type of impurities affecting a catalytic reaction varies depending on the types of reaction and catalyst, and until now, it has not been clarified what impurities have an adverse effect on a hydration reaction of an olefin using a heteropolyacid catalyst.

It is an object of the present invention to provide a method which enables the stable use of a catalyst for a long period of time in the production of an alcohol by a hydration reaction of an olefin using a heteropolyacid catalyst.

### [SOLUTION TO PROBLEM]

As a result of intensive studies, the present inventors have found that an aldehyde compound has a large effect on deterioration of a catalyst, particularly coking, in the production of an alcohol by a hydration reaction of an olefin using a heteropolyacid catalyst. Accordingly, it has been confirmed that a catalyst can be stably used for a long period of time by using a raw material having a small aldehyde compound content in a hydration reaction of an olefin using a heteropolyacid catalyst, and thus the present invention has been completed.

That is, the present invention relates to the following [1] to [7].
[1] A method for producing an alcohol comprising supplying water and an olefin having 2 to 5 carbon atoms to a reactor, and subjecting them to a hydration reaction in a gas phase using a solid acid catalyst on which a heteropolyacid or a salt thereof is supported, wherein an aldehyde compound content in a raw material mixture supplied to the reactor is 70 mol ppm or less.
[2] The method for producing an alcohol according to [1], wherein at least one selected from the group consisting of unreacted water, an unreacted olefin having 2 to 5 carbon atoms, and an ether compound by-produced by the reaction is supplied again to the reactor as a recycled raw material.
[3] The method for producing an alcohol according to [1] or [2], wherein the aldehyde compound contained in the recycled raw material is removed.
[4] The method for producing an alcohol according to [2] or [3], wherein at least one selected from the group consisting of gas absorption, adsorption, distillation, and reaction conversion is used as a means for removing the aldehyde compound from the recycled raw material.
[5] The method for producing an alcohol according to any one of [1] to [4], wherein silica is used as a carrier of the solid acid catalyst.
[6] The method for producing an alcohol according to any one of [1] to [5], wherein the heteropolyacid is at least one compound selected from the group consisting of silicotungstic acid, phosphotungstic acid, phosphomolybdic acid, silicomolybdic acid, silicovanadotungstic acid, phosphovanadotungstic acid, and phosphovanadomolybdic acid.
[7] The method for producing an alcohol according to any one of [1] to [6], wherein the olefin having 2 to 5 carbon atoms is ethylene and the alcohol produced by the hydration reaction is ethanol.

### [ADVANTAGEOUS EFFECTS OF INVENTION]

According to the present invention, in the production of an alcohol by a hydration reaction of an olefin using a heteropolyacid catalyst, coking of the heteropolyacid catalyst is suppressed, and the catalyst can be stably used over a long period of time.

### BRIEF DESCRIPTION OF DRAWINGS

[Fig. 1] A diagram showing an example of an alcohol production process to which the present invention can be applied.
[Fig. 2] A graph showing the reaction time and the change in the conversion rate of a raw material, ethylene, in the examples and comparative examples.
[Fig. 3] A graph showing the relationship between the reaction time and the catalyst layer peak temperature in the examples and comparative examples.
[Fig. 4] A graph showing the reaction time and the changes in the ethanol and diethyl ether selectivities in the examples and comparative examples.
[Fig. 5] A graph showing the reaction time and the change in the by-produced butene selectivity in the examples and comparative examples.
[Fig. 6] A graph showing the reaction time and the change in the by-produced acetaldehyde selectivity in the examples and comparative examples.

### DESCRIPTION OF EMBODIMENTS

Hereinafter, preferred embodiments of the present invention will be described, but it should be understood that the present invention is not limited to these embodiments only, and various applications can be made within the spirit and practice of the present invention.

### (Heteropolyacid catalyst)

A heteropolyacid catalyst according to one embodiment refers to a catalyst comprising a heteropolyacid or a salt thereof as a major active component of the catalyst.

### (Heteropolyacid or salt thereof)

A heteropolyacid is composed of a central element and a peripheral element to which oxygen is bonded. The central element is usually silicon or phosphorus, but may be any one selected from a wide variety of elements of Groups 1 to 17 of the Periodic Table of the Elements. Specific examples of the central element include a cupric ion; divalent ions of beryllium, zinc, cobalt and nickel; trivalent ions of boron, aluminum, gallium, iron, cerium, arsenic, antimony, phosphorus, bismuth, chromium and rhodium; tetravalent ions of silicon, germanium, tin, titanium, zirconium, vanadium, sulfur, tellurium, manganese, nickel, platinum, thorium, hafnium, cerium and other tetravalent rare earth ions; pentavalent ions of phosphorus, arsenic, vanadium, and antimony; a hexavalent ion of tellurium; and a heptavalent ion of iodine, but are not limited thereto. Specific examples of the peripheral element include tungsten, molybdenum, vanadium, niobium, and tantalum, but are not limited thereto.

Such heteropolyacids are also known as "polyoxoanions", "polyoxometalates" or "metal oxide clusters". The structures of some of the wellknown anions are named after the researchers in this field, and for example, the Keggin structure, the Wells-Dawson structure and the Anderson-Evans-Perl off structure are known. Details are described in "Chemistry of Polyacids" (edited by the Chemical Society of Japan, Quarterly Chemical Review No. 20, 1993). A heteropolyacid usually has a high molecular weight, e.g., a molecular weight in the range of 700 to 8,500, and includes not only a monomer thereof but also a dimeric complex thereof.

The salt of the heteropolyacid is not particularly limited as long as it is a metal salt or an onium salt in which some or all of the hydrogen atoms of the aforementioned heteropolyacid are substituted. Specific examples of the salt include metal salts of lithium, sodium, potassium, cesium, magnesium, barium, copper, gold and gallium, and onium salts of ammonia, etc., but are not limited thereto.

A heteropolyacid has relatively high solubility in polar solvents, such as water or other oxygenated solvents, particularly when the heteropolyacid is in the form of a free acid or some types of salts. The solubility of these salts can be controlled by selecting an appropriate counterion.

Examples of the heteropolyacid that can be used in the catalyst include:
silicotungstic acid: H₄[SiW₁₂O₄₀]·xH₂O
phosphotungstic acid: H₃[PW₁₂O₄₀]·xH₂O
phosphomolybdic acid: H₃[PMo₁₂O₄₀]·xH₂O
silicomolybdic acid: H₄[SiMo₁₂O₄₀]·xH₂O
silicovanadotungstic acid: H₄₊ₙ[SiVₙW₁₂₋ₙO₄₀]·W₂O
phosphovanadotungstic acid: H₃₊ₙ[PVₙW₁₂₋ₙO₄₀]·xH₂O
phosphovanadomolybdic acid: H₃₊ₙ[PVₙMo₁₂₋ₙO₄₀]·xH₂O
silicovanadomolybdic acid: H₄₊ₙ[SiVₙMo₁₂₋ₙO₄₀]·xH₂O
silicomolybdotungstic acid: H₄[SiMoₙW₁₂₋ₙO₄₀]·xH₂O
phosphomolybdotungstic acid: H₃[PMoₙW₁₂₋ₙO₄₀]·xH₂O
wherein n is an integer of 1 to 11 and x is an integer greater than or equal to 1, but are not limited thereto.

The heteropolyacid is preferably silicotungstic acid, phosphotungstic acid, phosphomolybdic acid, silicomolybdic acid, silicovanadotungstic acid, phosphovanadotungstic acid, or phosphovanadomolybdic acid, and more preferably silicotungstic acid, phosphotungstic acid, silicovanadotungstic acid, or phosphovanadotungstic acid.

There is no particular limitation on the method for synthesizing such a heteropolyacid, and any methods may be used. For example, a heteropolyacid can be obtained by heating an acidic aqueous solution (approximately pH1 to pH2) containing a salt of molybdic acid or tungstic acid and a simple oxoacid of a heteroatom or a salt thereof. A heteropolyacid compound can be isolated, for example, by crystallization separation as a metal salt from the produced aqueous heteropolyacid solution. Specific examples of the manufacture of the heteropolyacid are described on page 1413 of "New Experimental Chemistry 8, Synthesis of Inorganic Compound (III)" (edited by the Chemical Society of Japan, published by Maruzen Co., Ltd., August 20, 1984, third edition), but are not limited thereto. The structural confirmation of the synthesized heteropolyacid can be carried out by chemical analysis, as well as X-ray diffraction, UV, or IR measurements.

Preferred examples of the salt of the heteropolyacid include lithium salts, sodium salts, potassium salts, cesium salts, magnesium salts, barium salts, copper salts, gold salts, gallium salts, and ammonium salts of the aforementioned preferred heteropolyacids.

Specific examples of the salt of the heteropolyacid include a lithium salt of silicotungstic acid, a sodium salt of silicotungstic acid, a cesium salt of silicotungstic acid, a copper salt of silicotungstic acid, a gold salt of silicotungstic acid, a gallium salt of silicotungstic acid; a lithium salt of phosphotungstic acid, a sodium salt of phosphotungstic acid, a cesium salt of phosphotungstic acid, a copper salt of phosphotungstic acid, a gold salt of phosphotungstic acid, a gallium salt of phosphotungstic acid; a lithium salt of phosphomolybdic acid, a sodium salt of phosphomolybdic acid, a cesium salt of phosphomolybdic acid, a copper salt of phosphomolybdic acid, a gold salt of phosphomolybdic acid, a gallium salt of phosphomolybdic acid; a lithium salt of silicomolybdic acid, a sodium salt of silicomolybdic acid, a cesium salt of silicomolybdic acid, a copper salt of silicomolybdic acid, a gold salt of silicomolybdic acid, a gallium salt of silicomolybdic acid; a lithium salt of silicovanadotungstic acid, a sodium salt of silicovanadotungstic acid, a cesium salt of silicovanadotungstic acid, a copper salt of silicovanadotungstic acid, a gold salt of silicovanadotungstic acid, a gallium salt of silicovanadotungstic acid; a lithium salt of phosphovanadotungstic acid, a sodium salt of phosphovanadotungstic acid, a cesium salt of phosphovanadotungstic acid, a copper salt of phosphovanadotungstic acid, a gold salt of phosphovanadotungstic acid, a gallium salt of phosphovanadotungstic acid; a lithium salt of phosphovanadomolybdic acid, a sodium salt of phosphovanadomolybdic acid, a cesium salt of phosphovanadomolybdic acid, a copper salt of phosphovanadomolybdic acid, a gold salt of phosphovanadomolybdic acid, a gallium salt of phosphovanadomolybdic acid; a lithium salt of silicovanadomolybdic acid, a sodium salt of silicovanadomolybdic acid, a cesium salt of silicovanadomolybdic acid, a copper salt of silicovanadomolybdic acid, a gold salt of silicovanadomolybdic acid, and a gallium salt of silicovanadomolybdic acid.

The salt of the heteropolyacid is preferably a lithium salt of silicotungstic acid, a sodium salt of silicotungstic acid, a cesium salt of silicotungstic acid, a copper salt of silicotungstic acid, a gold salt of silicotungstic acid, a gallium salt of silicotungstic acid; a lithium salt of phosphotungstic acid, a sodium salt of phosphotungstic acid, a cesium salt of phosphotungstic acid, a copper salt of phosphotungstic acid, a gold salt of phosphotungstic acid, a gallium salt of phosphotungstic acid; a lithium salt of silicovanadotungstic acid, a sodium salt of silicovanadotungstic acid, a cesium salt of silicovanadotungstic acid, a copper salt of silicovanadotungstic acid, a gold salt of silicovanadotungstic acid, a gallium salt of silicovanadotungstic acid; a lithium salt of phosphovanadotungstic acid, a sodium salt of phosphovanadotungstic acid, a cesium salt of phosphovanadotungstic acid, a copper salt of phosphovanadotungstic acid, a gold salt of phosphovanadotungstic acid, or a gallium salt of phosphovanadotungstic acid.

As the salt of the heteropolyacid, it is particularly preferable that a lithium salt of silicotungstic acid, a cesium salt of silicotungstic acid, a lithium salt of phosphotungstic acid or a cesium salt of phosphotungstic acid be used.

### (Carrier)

The heteropolyacid catalyst can be used as it is, but is preferably used as supported on a carrier. The carrier is preferably at least one selected from the group consisting of silica, diatomaceous earth, titania, activated carbon, alumina, and silica alumina, and more preferably silica.

The shape of the carrier is not particularly limited. Examples of the shape include a spherical shape, a cylindrical shape, a hollow cylindrical shape, a plate shape, an elliptical shape, a sheet shape, and a honeycomb shape. The shape is preferably spherical, cylindrical, hollow cylindrical, or elliptical, and more preferably spherical or cylindrical, in order to facilitate filling into the reactor and supporting of a catalytically active component.

Although the size of the carrier is not particularly limited, it is desirable that the size be determined by taking into consideration handling at the time of producing a solid acid catalyst on which a catalytically active component is supported or at the time of filling the catalyst, the differential pressure after filling it into a reactor, the reaction performance of the catalytic reaction, etc., since they are affected by the size. The size of the carrier is preferably 1 mm to 20 mm, and more preferably 2 mm to 10 mm, when used in a fixed bed system.

Although there is no limitation on the strength of the carrier, the crush strength of the carrier is preferably 5 N or more, and more preferably 10 N or more, since cracking or breakage of a solid acid catalyst causes an increase in the differential pressure of a reactor or occlusion of a pipe. In the present disclosure, the crush strength is a value obtained when a load is applied to a carrier by using a digital hardness meter KHT-40N type manufactured by FUJIWARA SCIENTIFIC CO., LTD., and the carrier is crushed.

Although there is no limitation on the specific surface area of the carrier, the specific surface area by the BET method is preferably 50 m²/g or more, and more preferably 100 m²/g or more, since the activity of the catalyst increases as the specific surface area increases.

There is no particular limitation on the method for supporting the heteropolyacid or the salt thereof on the carrier. In general, it can be carried out by making the carrier absorb a solution or suspension obtained by dissolving or suspending the heteropolyacid or the salt thereof in a solvent and evaporating the solvent.

The amount of the heteropolyacid or the salt thereof to be supported on the carrier can be adjusted, for example, by dissolving the heteropolyacid or the salt thereof in an amount of distilled water that corresponds to the amount of water absorbed by the carrier, and impregnating the carrier with the solution. In another embodiment, the amount of the heteropolyacid or the salt thereof to be supported on the carrier can also be adjusted by immersing the carrier in a solution of an excess amount of the heteropolyacid or the salt thereof with moderate movement, followed by filtration to remove an excess heteropolyacid or salt thereof. The volume of the solution or suspension varies depending on the carrier used, the supporting method, etc. By placing a carrier in which the heteropolyacid or the salt thereof is impregnated in a heating oven for several hours to evaporate a solvent, a solid acid catalyst supported on the carrier can be obtained. The drying method is not particularly limited, and various methods, such as a stationary method, and a belt conveyor method, can be used. The amount of the heteropolyacid or the salt thereof supported on the carrier can be accurately measured by chemical analysis, such as ICP and XRF

The amount of the heteropolyacid or the salt thereof supported on the carrier is preferably 10 to 300 parts by mass, and more preferably 20 to 200 parts by mass, in terms of the total mass of the heteropolyacid and the salt thereof with respect to 100 parts by mass of the carrier.

### (Method for producing alcohol by hydration reaction of olefin)

Next, a method for producing an alcohol by a hydration reaction of an olefin using a heteropolyacid catalyst will be described. An alcohol can be obtained by supplying water and an olefin having 2 to 5 carbon atoms to a reactor and subjecting them to a hydration reaction in a gas phase using a solid acid catalyst on which the heteropolyacid or the salt thereof is supported.

A specific example of the alcohol production reaction by the hydration reaction of an olefin having 2 to 5 carbon atoms is represented by Formula (1): 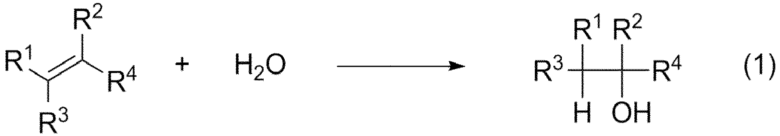 wherein R¹ to R⁴ each independently represent a hydrogen atom or an alkyl group having 1 to 3 carbon atoms, and the sum of the carbon atoms of R¹ to R⁴ is 0 to 3.

There is no particular limitation on the olefin having 2 to 5 carbon atoms which can be used in the hydration reaction of an olefin using the heteropolyacid catalyst. The olefin having 2 to 5 carbon atoms is preferably ethylene, propylene, n-butene, isobutene, pentene or a mixture of two or more thereof. Among them, ethylene is more preferable. Although there is no limitation on the use ratio of the olefin and water, the molar ratio of the olefin to water is preferably water/olefin = 0.01 to 2.0, and more preferably water/olefin = 0.1 to 1.0, since the concentration dependence of the olefin on the reaction rate is large and the energy cost of the alcohol production process increases when the water concentration is high.

There is no limitation on the mode of the hydration reaction of an olefin using the heteropolyacid catalyst, and any of the reaction modes can be used. From the viewpoint of ease of separation from the catalyst and reaction efficiency, the preferred examples of the mode include a fixed-bed, a fluidized-bed, and a suspension-bed. A fixed-bed that requires the least energy for separation from the catalyst is more preferable.

The gas space velocity in the reactor in the case of using a fixed-bed is not particularly limited, but is preferably 500 to 15,000/hr, and more preferably 1000 to 10,000/hr, from the viewpoint of energy and reaction efficiency. When the gas space velocity is 500/hr or more, the amount of the catalyst used can be effectively reduced, and when the gas space velocity is 15000/hr or less, the amount of gas circulation can be reduced, so that the production of alcohol can be more efficiently carried out within the above ranges.

There is no limitation on the reaction pressure in the hydration reaction of an olefin using the heteropolyacid catalyst. Since the hydration reaction of an olefin is a reaction in which the number of molecules decreases, it is generally advantageous to proceed at high pressure. The reaction pressure is preferably 0.5 to 7.0 MPaG, and more preferably 1.5 to 4.0 MPaG. "G" means a gauge pressure. When the reaction pressure is 0.5 MPaG or more, a satisfactory reaction rate can be obtained, and when the reaction pressure is 7.0 MPaG or less, it is possible to further reduce costs regarding installation of equipment as countermeasures for condensation of an olefin and in relation to evaporation of an olefin, equipment for high pressure gas safety, and energy.

The reaction temperature of the hydration reaction of an olefin using the heteropolyacid catalyst is not particularly limited, and the reaction can be carried out at a wide range of temperatures. In view of the thermal stability of the heteropolyacid or the salt thereof and the temperature at which water, one of the raw materials, does not condense, the preferred reaction temperature is 100 to 550°C, and more preferably 150 to 350°C.

The hydration reaction of an olefin using the heteropolyacid catalyst is an equilibrium reaction, and the conversion rate of olefin will be an equilibrium conversion rate at most. For example, the equilibrium conversion rate in the production of ethanol by the hydration of ethylene is calculated to be 7.5% at a temperature of 200°C and a pressure of 2.0 MPaG. Therefore, in the method for producing an alcohol by the hydration of an olefin, a maximum conversion rate is determined by the equilibrium conversion rate, and as can be seen in an example of ethylene, the hydration reaction of an olefin tends to have a small equilibrium conversion rate, and thus it is strongly required in the industry to carry out the hydration reaction of an olefin with high efficiency under mild conditions.

In the hydration reaction of an olefin using the heteropolyacid catalyst, loss of the olefin can be reduced by recycling any unreacted olefin into a reactor. There is no limitation on the method for recycling the unreacted olefin into the reactor, and the olefin may be isolated and recycled from a process fluid coming out of the reactor, or may be recycled together with other inert components. Typically, an industrial grade olefin often contains a very small amount of paraffin. Therefore, for example, when ethylene containing ethane is used and the unreacted ethylene is recycled to the reactor, it is desirable to purge a portion of the recovered ethylene gas out of the system, in order to prevent concentration and accumulation of ethane.

In the hydration reaction of an olefin using the heteropolyacid catalyst, the produced alcohol may be dehydrated to generate an ether compound as a by-product. For example, when ethanol is obtained by the hydration of ethylene, diethyl ether is by-produced. It is considered that this diethyl ether is generated by a dehydration reaction of two molecules of ethanol, and when ethanol is produced by the hydration reaction of ethylene, the reaction yield is remarkably lowered. However, by recycling the by-produced diethyl ether into the reactor, diethyl ether is converted into ethanol, so that ethanol can be produced from ethylene with extremely high efficiency. Although there is no particular limitation on the method for recycling the by-produced ether compound into the reactor, there are, for example, a method including isolating an ether compound from components distilled from the reactor and recycling the ether compound into the reactor, and a method including recycling the ether compound into the reactor as a gas component together with an unreacted olefin.

In the hydration reaction of an olefin using the heteropolyacid catalyst, the produced alcohol in a state of being dissolved in a large amount of water which has not been converted as a reaction raw material is sent to a separation and purification step together with other by-products. In the separation and purification step, the alcohol, water, and the other by-products are separated, and an alcohol having a purity equal to or higher than a certain level by the purification is obtained as a product. At this time, the water simultaneously obtained may be disposed of as waste water, but from the viewpoint of impact or load on the environment, it is desirable to recycle it in the process and use it again as a raw material for the reaction. There is no limitation on the type and number of apparatuses in the separation and purification step, and a distillation apparatus or a membrane separation apparatus may be used, and different apparatuses can be used in combination if necessary.

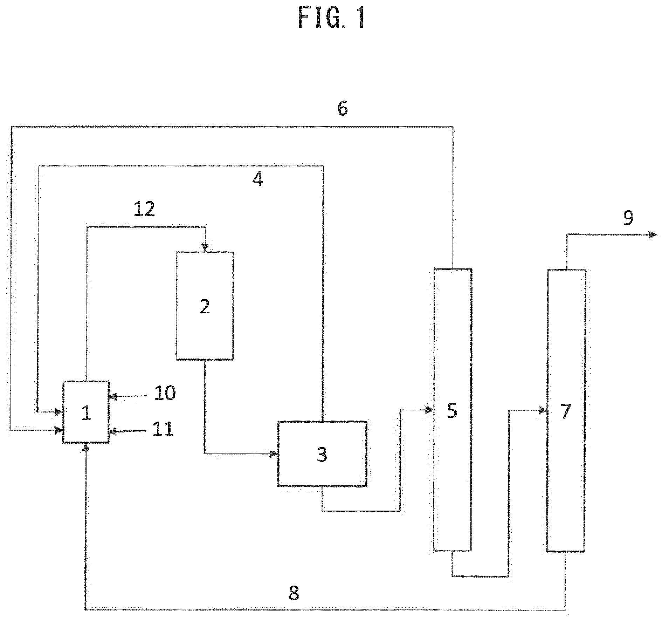

An example of a production process using a recycled raw material is shown in FIG. 1. The present invention is not limited by the flow of FIG. 1. In the process flow of FIG. 1, an unreacted recycled olefin gas 4, unreacted recycled water 8, and by-produced recycled ether 6 together with a raw material olefin gas 10 and raw material water 11 are supplied to an evaporator 1, mixed and gasified, and supplied to a reactor 2 as a raw material mixture 12. From the reaction product, an unreacted olefin gas is separated in an intermediate tank 3 and is supplied to the evaporator 1 as a recycled olefin gas 4. It is preferable that a portion of the recycled olefin gas 4 be discharged (purged) out of the system as described above. In a low boiling point component removal tower 5, a by-produced ether compound which is a low boiling point component is separated from the top of the tower by distillation or the like, and is returned to the evaporator 1 as a recycled ether 6, which is then included in the reaction raw material. A high boiling point component drawn out from the bottom of the low boiling point component removal tower 5 is distilled and separated into a crude alcohol 9 which is a low boiling point component as a target component and water which is a high boiling point component in a water removal tower 7. Water drawn out from the bottom of the water removal tower 7 is returned to the evaporator 1 as recycled water 8. If necessary, the crude alcohol 9 can be further purified to obtain a product alcohol.

In the hydration reaction of an olefin using the heteropolyacid catalyst, it is possible to reuse an olefin, a by-produced ether compound, and water as a recycled raw material, and also to use a by-product of another production process as a raw material, as described above. There is no limitation on the source of these raw materials.

In studying the use of a plurality of raw materials, the present inventors have found that impurities mixed in a raw material affect the reaction and the state of a solid acid catalyst depending on the types of the impurities. In particular, an aldehyde compound has high polymerization reactivity and polymerizes on a solid acid catalyst surface to form a coke, which reduces the reaction activity of the catalyst or deteriorates the selectivity thereof. The aldehyde compound is supplied to a reactor as an impurity in a raw material or a reaction by-product contained in a recycled raw material. Examples of the aldehyde compound include acetaldehyde, butyraldehyde, crotonaldehyde, and hexanal. It is presumed that the aldehyde compound of the reaction by-product is by-produced by dehydrogenation of an alcohol compound. For example, it is considered that acetaldehyde is by-produced from ethanol and butyraldehyde is by-produced from butanol.

In order to stably use the heteropolyacid catalyst for a long period of time, the total concentration of aldehyde compound(s) in a raw material mixture supplied to a reactor (hereinafter, referred to as "reactor inlet concentration of the aldehyde compound(s)") needs to be 70 mol ppm or less, and is preferably 50 mol ppm or less. When a plurality of kinds of aldehyde compounds are present, the total content thereof serves as a reference for the total concentration of the aldehyde compounds.

There is no limitation on the means for lowering the reactor inlet concentration of the aldehyde compound(s). For example, a raw material (also including a recycled raw material) and an aldehyde compound may be separated using a separation and purification apparatus, and a raw material having a reduced aldehyde concentration may be returned to a supply, or a plurality of raw materials may be appropriately combined so that the aldehyde concentration does not exceed a certain value.

Although there is no limitation on the means for separating the raw material and the aldehyde compound, at least one selected from the group consisting of gas absorption, adsorption, distillation, and reaction conversion can be used. For the separation of the raw material and the aldehyde compound, a distillation tower, an absorption tower, an adsorption apparatus, a membrane separation apparatus or the like may be used. Since the raw material contains a plurality of types of substances, such as an olefin, an ether compound, and water, a different treatment may be applied to each individual raw material. For example, in the absorption tower, the aldehyde compound contained in the olefin can be removed by absorbing it in water, while the aldehyde compound contained in recycled water can also be removed by absorbing it in a hydrocarbon compound or a solvent. In other words, an efficient separation means can be appropriately selected based on the type and properties of the raw material and the type and properties of the aldehyde compound contained in the raw material.

### EXAMPLES

Although the present invention will be further described with reference to the following Examples and Comparative Examples, these examples illustrate the summary of the present invention, and the present invention is not limited to these examples.

### 1. Preparation of silica carrier

25 parts by mass of fumed silica F-1, 75 parts by mass of silica gel S-1, and 45 parts by mass of colloidal silica C-1 (9 parts by mass in terms of solid content) were kneaded by a kneader, and then water and additives (10 parts by mass of methyl cellulose: METOLOSE ^{®} SM-4000 manufactured by Shin-Etsu Chemical Co., Ltd., and 5 parts by mass of a resin-based binder: Cerander ^{®} YB-132A manufactured by Yuken Industry Co., Ltd.) were added in an appropriate amount, with the status of a mixture being monitored, and the mixture was further kneaded to obtain a kneaded material. Next, the kneaded material was put into an extruder, to which a die having a circular hole of 6 mmcp was attached, the kneaded material was extruded, and the extruded intermediate material was subjected to extrusion molding while cutting with a cutter so that the extruded intermediate material had the same length as the diameter of the circular hole used. The obtained shaped body before calcination was formed into a spherical shape by Marumerizer ^{®}, and then dried at 70°C for 24 hours or more, and calcined at about 820°C under an air atmosphere, and cooled to obtain a silica carrier A.

### 2. Preparation of solid acid catalyst in which heteropolyacid is supported on silica carrier

In a 100 mL beaker, 40.7 g of a commercially available Keggin silicotungstic acid 26 hydrate (H₄SiW₁₂O₄₀·26H₂O; manufactured by Nippon Inorganic Colour & Chemical Co., Ltd.) was weighed, a small amount of distilled water was added to solve silicotungstic acid, and then the solution was transferred to a 200 mL graduated cylinder. Then, distilled water was added so that the liquid amount of the silicotungstic acid solution in the graduated cylinder was 95% of the water absorption rate of a carrier to be used, and the mixture was stirred so that the entire mixture was uniform. After the stirring, the aqueous solution of silicotungstic acid was transferred to a 200 mL volumetric flask, and then weighed 100 mL of the silica carrier A was put into the 200 mL volumetric flask, and the contents of the volumetric flask were mixed so that the aqueous solution of silicotungstic acid contacted the entire carrier. The silica carrier A on which silicotungstic acid was supported was transferred to a porcelain dish, air-dried for one hour, and then dried for 5 hours in a hot air dryer adjusted to 150°C. After the drying, the carrier was transferred into a desiccator and cooled to room temperature to obtain a solid acid catalyst A.

### 3. Hydration reaction of ethylene

A reactor filled with a predetermined amount of the solid acid catalyst was controlled to reach a predetermined temperature and to be pressurized to a predetermined pressure, and a predetermined amount of water vaporized by an evaporator and a predetermined amount of ethylene from a mass flow controller were introduced into the reactor. A reaction gas after passing through the reactor was cooled, and a condensed liquid and the reaction gas from which the condensate was removed were sampled for a certain period of time, respectively. The sampled liquid (reaction liquid) and the reaction gas were analyzed using a gas chromatography analyzer and a Karl Fischer analyzer to calculate the reaction results.

### 4. Analysis of reaction gas

The sampled gas was analyzed by using a gas chromatography apparatus (apparatus name: 7890) manufactured by Agilent Technologies Japan, Ltd., and a system program based on a plurality of columns and two detectors.

### Gas chromatography conditions:

Oven: kept at 40°C for 3 minutes, then raised to 200°C at 20°C/min
Carrier gas: helium
Split ratio: 10:1

### Columns used: manufactured by Agilent Technologies Japan, Ltd.

HP-1: 2 m
GasPro: 30 m × 320 µm
DB-624: 60 m × 320 µm × 1.8 µm

### Detectors:

Front detector: FID (heater: 230°C, hydrogen flow rate 40 mL/min, air flow rate 400 L/min)
Back detector: FID (heater: 230°C, hydrogen flow rate 40 mL/min, air flow rate 400 L/min)
Aux detector: TCD (heater: 230°C, reference flow rate 45 mL/min, make-up flow rate 2 mL/min)

### 5. Analysis of reaction solution

The sampled reaction solution was analyzed by using a gas chromatography apparatus (apparatus name: 6850) manufactured by Agilent Technologies Japan, Ltd. Further, the concentration of water in the reaction solution was analyzed by a Karl Fischer analyzer manufactured by Mitsubishi Chemical Co., Ltd.
Columns used: PoraBOND Q 25 m × 0.53 mm ID × 10 µM
Oven temperature: kept at 100°C for 2 minutes, then raised to 240°C at 5°C/min.
Injection temperature: 250°C
Detector temperature: 300°C

### 6. Analysis of deposited carbon amount in used catalyst

The catalyst used in the reaction was pulverized to form a powder sample, which was analyzed by using a simultaneous CHN analyzer MT-6 manufactured by Yanaco Technical Science Co., Ltd., to quantify the amount of deposited carbon.

### <Example 1>

4 mL of the solid acid catalyst A was weighed, and filled in a tubular reactor (made from SUS316, inner diameter 10 mm, length 300 mm), which was then pressurized to 0.75 MPaG after nitrogen gas replacement. Then, the reactor was heated to 160°C, and at a stage where the temperature was stable, amounts of water and ethylene so that the molar ratio of water to ethylene was 0.3 were fed to the reactor at a GHSV (gas space velocity) of 4000/hr to carry out a hydration reaction of ethylene. After the feed of water and ethylene, the temperature of the reactor was adjusted so that the peak temperature of the catalyst layer reached 190°C after the temperature was stable. At 2 hours after the peak temperature was stabilized at 190°C, a gas passed through the reactor was cooled, and sampling of a condensed reaction solution and a reaction gas from which the condensate was removed was carried out for 1 hours. Reaction results of the catalyst were calculated based on the masses of the obtained condensate and reaction gas, the gas flow rates, and analysis results. From the second day onwards, the test was continued while adjusting the temperature of the reactor so that the ethylene conversion rate was 6%.

### <Example 2>

The reaction was carried out in the same manner as in Example 1, except that an aqueous acetaldehyde solution adjusted so that the reactor inlet concentration of acetaldehyde was 50 mol ppm was used as a raw material instead of water, from the second day of the reaction test.

### <Comparative Example 1>

The reaction was carried out in the same manner as in Example 1, except that an aqueous acetaldehyde solution adjusted so that the reactor inlet concentration of acetaldehyde was 100 mol ppm was used as a raw material instead of water, from the second day of the reaction test.

### <Reaction results and deposited carbon amount>

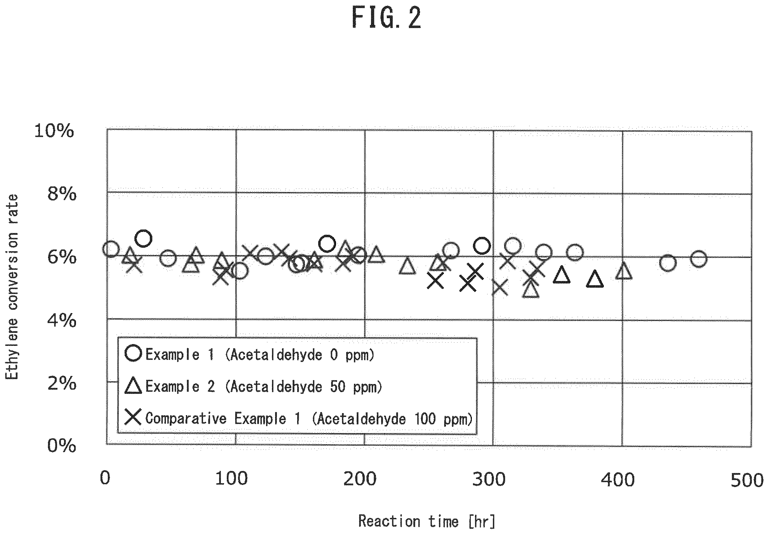

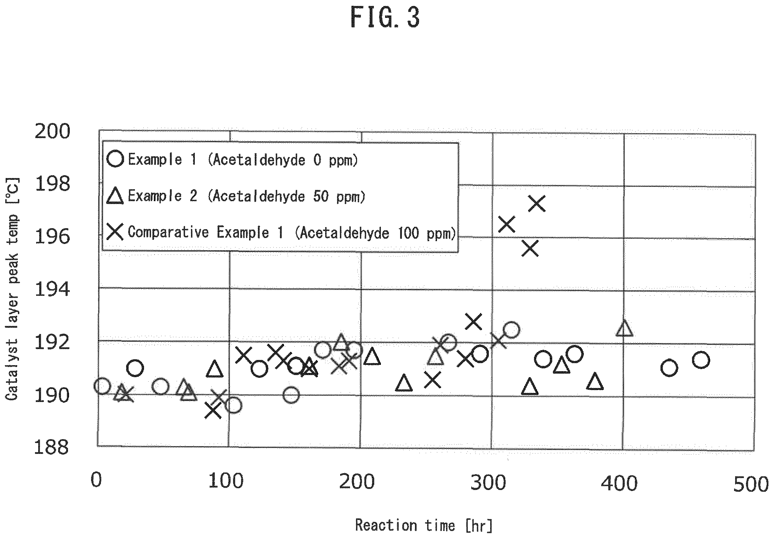

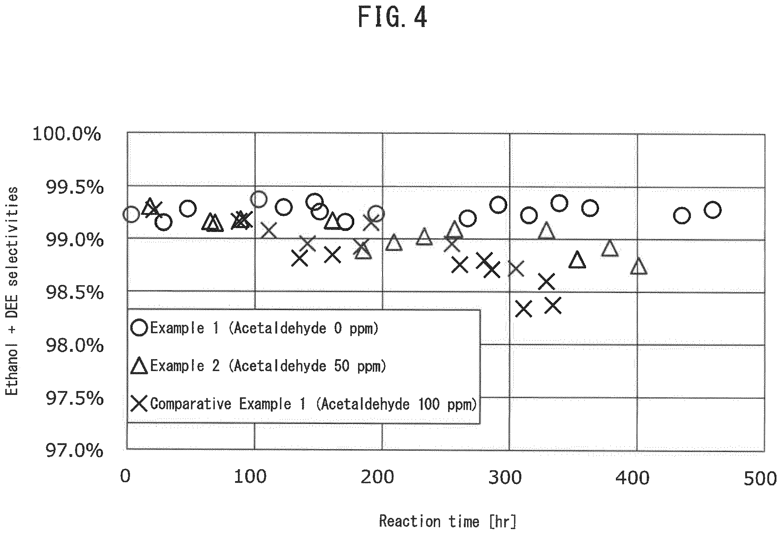

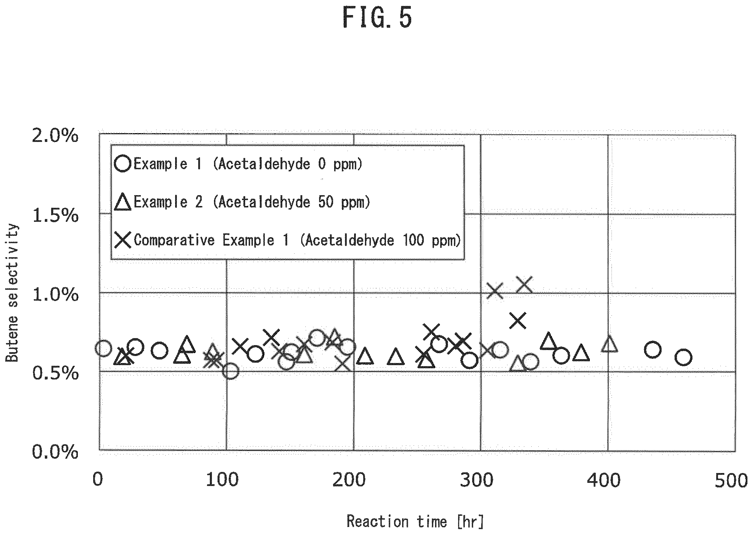

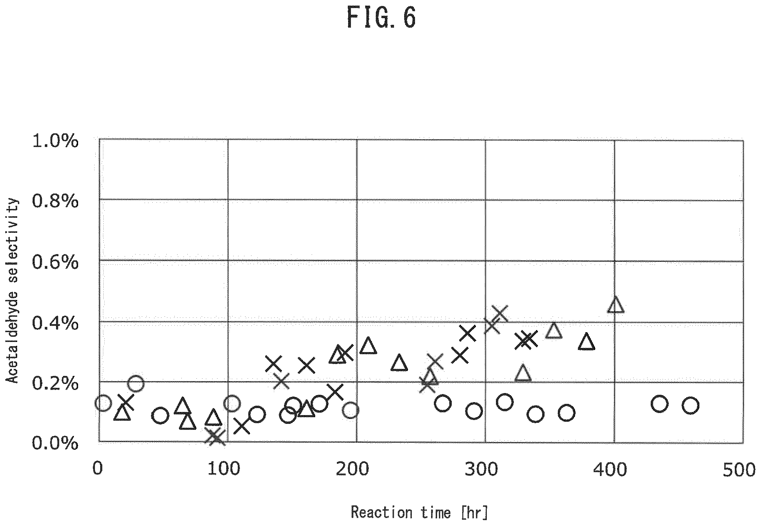

The reaction results of Example 1, Example 2 and Comparative Example 1 are shown in FIGs. 2 to 6. In Examples 1 and 2, an ethylene conversion rate of 6% could be maintained by increasing the catalyst layer peak temperature by about 1°C over 400 hours. On the other hand, in Comparative Example 1, it can be understood that an ethylene conversion rate of 6% could not be maintained unless the catalyst layer peak temperature was increased by 5°C or more after 300 hours, and the catalytic activity was lowered. The selectivities of butene and acetaldehyde, by-products, also worsened (increased). In Example 2, although the selectivity of acetaldehyde increased, the butene selectivity did not increase, and by increasing the catalyst layer peak temperature by about 1°C, an ethylene conversion rate of 6% could be maintained, as described above.

Table 1 shows the amount of deposited carbon in the used catalyst. In Comparative Example 1, despite the shortest reaction time, the amount of deposited carbon was larger than those of Examples 1 and 2, and it can be understood that the solid acid catalyst was deteriorated by coking.

**Table 1**

| Test | Reactor inlet concentration of acetaldehyde [mol ppm] | Reaction Time [hr] | Deposited carbon amount in used catalyst [% by mass] |
|---|---|---|---|
| Example 1 | 0 | 460 | 0.50 |
| Example 2 | 50 | 423 | 1.12 |
| Comparative Example 1 | 100 | 334 | 1.43 |

### INDUSTRIAL APPLICABILITY

The present invention is industrially useful in that, in the production of an alcohol by a hydration reaction of an olefin using a heteropolyacid catalyst, a catalyst can be stably used for a long period of time by reducing an aldehyde compound in a raw material, and a stable production amount of an alcohol can be secured.

### REFERENCE SIGNS LIST

1: Evaporator
2: Reactor
3: Intermediate tank
4: Recycled olefin gas
5: Low boiling point component removal tower
6: Recycled ether
7: Water removal tower
8: Recycled water
9: Crude alcohol
10: Raw material olefin gas
11: Raw material water
12: Raw material mixture

## Claims

1. A method for producing an alcohol comprising supplying water and an olefin having 2 to 5 carbon atoms to a reactor, and subjecting them to a hydration reaction in a gas phase using a solid acid catalyst on which a heteropolyacid or a salt thereof is supported, wherein an aldehyde compound content in a raw material mixture supplied to the reactor is 70 mol ppm or less.

2. The method for producing an alcohol according to claim 1, wherein at least one selected from the group consisting of unreacted water, an unreacted olefin having 2 to 5 carbon atoms, and an ether compound by-produced by the reaction is supplied again to the reactor as a recycled raw material.

3. The method for producing an alcohol according to claim 1 or 2, wherein the aldehyde compound contained in the recycled raw material is removed.

4. The method for producing an alcohol according to claim 2 or 3, wherein at least one selected from the group consisting of gas absorption, adsorption, distillation, and reaction conversion is used as a means for removing the aldehyde compound from the recycled raw material.

5. The method for producing an alcohol according to any one of claims 1 to 4, wherein silica is used as a carrier of the solid acid catalyst.

6. The method for producing an alcohol according to any one of claims 1 to 5, wherein the heteropolyacid is at least one compound selected from the group consisting of silicotungstic acid, phosphotungstic acid, phosphomolybdic acid, silicomolybdic acid, silicovanadotungstic acid, phosphovanadotungstic acid, and phosphovanadomolybdic acid.

7. The method for producing an alcohol according to any one of claims 1 to 6, wherein the olefin having 2 to 5 carbon atoms is ethylene and the alcohol produced by the hydration reaction is ethanol.
